# EUROPÄISCHE PATENTSCHRIFT

(11) **EP 3 153 185 B1**
(45) Veröffentlichungstag und Bekanntmachung des Hinweises auf die Patenterteilung: **25.03.2020**
(21) Anmeldenummer: 16196808.6
(22) Anmeldetag: 19.06.2013
(51) Int. Cl.: A61L 26/00, A61K 31/70

(54) **ZUSAMMENSETZUNG, ENTHALTEND GLUCOSYLGLYCEROL ZUR FÖRDERUNG DER WIEDERHERSTELLUNG VON VERLETZTEM KÖRPERGEWEBE**
COMPOSITION CONTAINING GLUCOSYLGLYCEROL FOR PROMOTING THE REGENERATION OF INJURED BODY TISSUE
COMPOSITION CONTENANT DU GLUCOSYLGLYCEROL DESTINÉE À FACILITER LA RÉGÉNÉRATION DE TISSUS CORPORELS ENDOMMAGÉS

(30) Priorität: 09.07.2012 DE 102012013482
(43) Veröffentlichungstag der Anmeldung: 12.04.2017
(62) Teilanmeldung aus: 13737554.9
(73) Patentinhaber: bitop AG, 58453 Witten (DE)
(72) Erfinder: BILSTEIN, Andreas, 50129 Bergheim (DE); SCHERNER, Olaf, 45134 Essen (DE); LENTZEN, Georg, 46483 Wesel (DE)
(74) Vertreter: Schöneborn, Holger

(56) Entgegenhaltungen:
- DE-A1- 19 540 749
- DE-A1-102007 052 380
- DE-A1-102008 039 231
- DATABASE WPI Week 200950 Thomson Scientific, London, GB; AN 2009-L73819 XP002761611, & JP 2009 161564 A (TOKIWA YAKUHIN KOGYO KK) 23. Juli 2009 (2009-07-23)
- DATABASE WPI Week 200501 Thomson Scientific, London, GB; AN 2005-002847 XP002761612, & JP 2004 331579 A (NOEVIR KK) 25. November 2004 (2004-11-25)

## Beschreibung

Die Erfindung betrifft eine Zusammensetzung, mit der die Wiederherstellung von verletztem Körpergewebe gefördert werden kann.

Verletzungen von Körpergewebe können auf unterschiedliche Art und Weise zustande kommen. Insbesondere können Gewebe durch äußere Einflüsse, d. h. auf traumatische Weise entstehen. Allgemein werden derartige Gewebeverletzungen, insbesondere im Bereich der Haut oder Schleimhaut, auch als Wunde bezeichnet. Neben Gewebeverletzungen, die auf äußere Einflüsse zurückzuführen sind, sind auch nicht traumatische Verletzungen als Ulcus (Geschwür) bekannt.

Für gewöhnlich beginnt die Wiederherstellung von geschädigtem Körpergewebe auf natürliche Weise bereits kurze Zeit nach der Verletzung. Im Fall einer Wunde setzt sehr bald die Blutgerinnung ein, so dass das zerstörte Blutgefäß durch einen Blutpfropfen (Gerinnsel) verschlossen wird. In der sich anschließenden Exsudationsphase tritt Wundsekret aus, wodurch Fremdkörper und Keime aus der Wunde herausbefördert werden. Das Immunsystem sorgt für die Abtötung von Bakterien.

In einer sich anschließenden Proliferationsphase entsteht neues Bindegewebe, sodass der durch die Wunde herbeigeführte Defekt ausgefüllt wird. Schließlich wird in einer Regenerationsphase die Wunde durch Überwachsen mit Epithelzellen verschlossen, die von intaktem Epithelgewebe im Bereich der Wundränder ausgehen.

Während die natürliche Wundheilung im Regelfall und bei nicht zu großen Wunden relativ problemlos verläuft, kann es bei großflächigen Wunden unter anderem aufgrund der starken Exsudatbildung zu Komplikationen kommen. Gleiches gilt für verschiedene Ulcera. Besonders problematisch sind darüber hinaus chronische Wunden, die auf eine dauerhafte Druckbelastung zurückzuführen sind (Dekubitus) oder auch eine verzögerte Wundheilung infolge von Diabetes mellitus. Letzterer führt bei manchen Patienten zum sog. diabetischen Fußsyndrom, das für 2/3 aller Amputationen in Deutschland verantwortlich ist.

Es stellt sich somit die Aufgabe, ein Mittel zur Verfügung zu stellen, mit dem die Wiederherstellung von verletztem Körpergewebe unterstützt und gefördert werden kann.

Diese Aufgabe wird erfindungsgemäß gelöst durch eine Zusammensetzung zur Verwendung nach Anspruch 1.

Glucosylglycerol, genauer 2-O-α-D-Glucosylglycerol, ist als Naturstoff bekannt und wird beispielsweise von Cyanobakterien synthetisiert, denen es als osmoprotektive Substanz dient. Auf diese Weise gestattet es den Cyanobakterien das Wachstum in salzhaltigen Medien mit einer Konzentration bis zu 1,5 M NaCl. Das Molekül wird in hohen Konzentrationen im Cytoplasma akkumuliert, um auf diese Weise den aufgrund der hohen Salzkonzentration in der Umgebung vorliegenden osmotischen Druck zu verringern und die Zelle vor Wasserverlust zu schützen. Ein Beispiel ist das Cyanobakterium *Synechocystis* sp. PCC 6803.

Darüber hinaus wird das Molekül auch von Pflanzen der Gattung Myrothamnus synthetisiert. Bei diesen Pflanzen handelt es sich um wechselfeuchte Pflanzen, *Myrothamnus flabellifolia* ist ein kleiner Strauch aus dem südlichen Afrika, der auf Felsplatten wächst und bis zu 60 Zentimeter hoch wird. Die hier auftretenden mehrere Monate andauernden Dürreperioden übersteht die Pflanze unbeschadet im ausgetrockneten Zustand. Sobald es jedoch regnet, wird die Pflanze innerhalb weniger Stunden wieder grün, woraus sich auch der Beiname "Auferstehungspflanze" ergibt. Im Bereich der Kosmetik und Dermatologie ist die Verwendung von Glucosylglycerol als Moisturizer bekannt (DE 195 40 749 A1). Daneben zeigt die DE 10 2008 039 231 A1 auch die Verwendung von Glucosylglycerolen zur Steigerung der Expression von Zellschutzenzymen. Die japanische Druckschrift JP 2009-161564 A offenbart die Verwendung von Glucosylglycerol zur Behandlung von atopischer Dermatitis. Die Struktur von 2-O-α-D-Glucosylglycerol ist wie folgt:

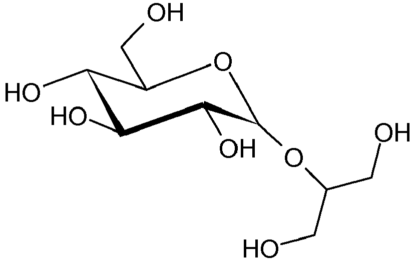

Bevorzugt handelt es sich bei dem Glucosylglycerol um das natürlich vorkommende 2-O-α-D-Glucosylglycerol, wie es beispielsweise von Cyanobakterien der Gattung *Synechocystis* akkumuliert wird. Entsprechende Wirkungen können jedoch auch bei β-glycosidischer Verknüpfung der Glucose mit dem Glycerin-Molekül oder bei Verknüpfung der Glucose mit dem Glycerin in 1-Position erwartet werden. Im Einzelnen ist somit neben der Verwendung des natürlichen Glucosylglycerols auch die Verwendung von 1-O-α-Glucosylglycerol, 1-O-β-Glucosylglycerol und 2-O-β-Glucosylglycerol in der D- und L-Konfiguration denkbar. Die einzelnen Moleküle (hier nur D-Konfiguration) sind im Folgenden dargestellt:

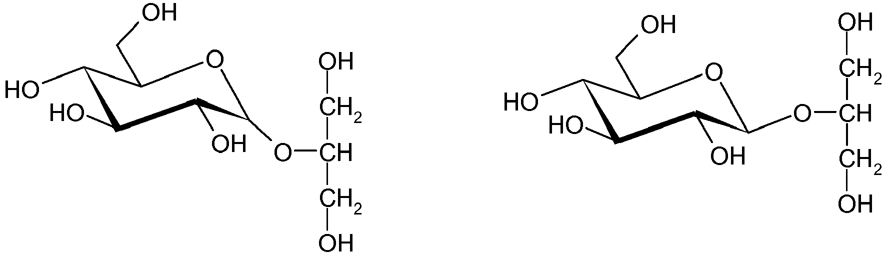

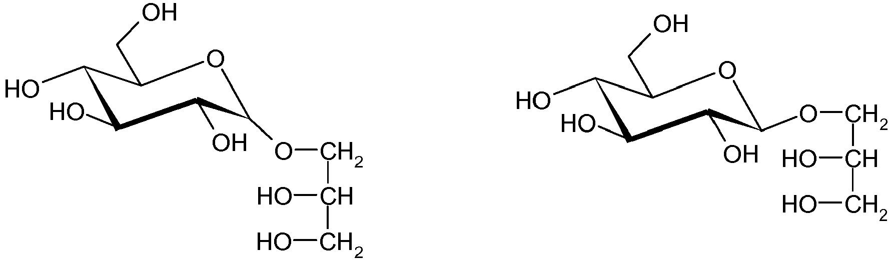

Bei dem Körpergewebe, dessen Wiederherstellung erfindungsgemäß durch die Glucosylglycerol enthaltenden Zusammensetzungen gefördert werden kann, kann es sich insbesondere um Haut oder Schleimhaut handeln. Die Verletzung kann insbesondere traumatischer Natur sein. Hierunter wird verstanden, dass die Verletzung durch äußere Einflüsse, beispielsweise Stöße, Schnitte, Stiche, Bisse oder ähnliches herbeigeführt wird. Derartige mechanisch verursachte Wunden können z. B. durch Unfälle oder im Rahmen von Operationen entstehen.

Im Falle von geschädigten Schleimhäuten spricht man auch von Mukositis, deren Behandlung ebenfalls unter die Förderung der Wiederherstellung von verletztem Körpergewebe gemäß Erfindung fällt. Eine Mukositis kann unterschiedliche Ursachen haben. Da Schleimhautzellen eine hohe Regenerationsrate aufweisen, tritt eine Mukositis beispielsweise häufig als Nebenwirkung im Rahmen der Krebsbehandlung durch Chemo- oder Strahlentherapie auf. Darüber hinaus sorgt ein geschwächtes Immunsystem, beispielsweise bei immunsupprimierten Patienten, für eine Zunahme von Infektionen, die ihrerseits zu Entzündungen der Schleimhaut führen können. Insbesondere können die Mundschleimhäute sowie die Schleimhäute des Verdauungssystems (Magen, Darm) betroffen sein.

Abgesehen von mechanisch verursachten Wunden existieren jedoch weitere Arten von Gewebeverletzungen, die ebenfalls mit Hilfe der erfindungsgemäßen Zusammensetzung behandelt werden können. Hierzu gehören beispielsweise thermische Wunden aufgrund von Verbrennungen, Verbrühungen oder Erfrierungen, Brandwunden, Verätzungen oder Wunden, die auf ionisierende Strahlung zurückzuführen sind.

Neben der Förderung der Wiederherstellung von verletztem Körpergewebe, wobei die Verletzung auf äußere Einflüsse zurückzuführen ist, ist die erfindungsgemäße Zusammensetzung auch zur Behandlung von Geschwüren (Ulcera) geeignet. Diese können unterschiedliche Ursachen haben, beispielsweise Durchblutungsstörungen, Tumore oder Infektionen. Mit Hilfe der erfindungsgemäßen Zusammensetzung behandelbare Geschwüre sind Ulcus cruris ("offenes Bein"), Dekubitus (Druckulcus), Malum perforans (Fußdruckgeschwür), Ulcus durum, Ulcus molle, Ulcus rodens und Ulcus corneae.

Besondere Bedeutung hat die Zusammensetzung bei der Behandlung von chronischen Verletzungen, insbesondere chronischen Wunden oder chronischen Ulcera. Ein Beispiel ist das diabetische Fußsyndrom (DFS), umgangssprachlich auch als diabetischer Fuß bezeichnet. Hierbei entstehen kleinere Verletzungen, insbesondere am Fuß oder am Unterschenkel, die an sich problemlos abheilen würden, aufgrund der schlechten Wundheilung bei Diabetes-Patienten jedoch häufig von Dauer sind. Die schlechte Wundheilung ist unter anderem auf bei Diabetes-Patienten auftretende Durchblutungsstörungen zurückzuführen. Bei dem diabetischen Fußsyndrom können sich Geschwüre tief in das Körperteil hinein ausbreiten, wobei zusätzlich das Risiko besteht, dass eine Infektion mit Keimen auftritt. Die Zahl der aufgrund des diabetischen Fußsyndroms jährlich notwendigen Amputationen ist erheblich, woraus sich die Notwendigkeit der Zurverfügungstellung einer wirkungsvollen Behandlungsmöglichkeit ergibt.

Eine weitere, häufig auftretende Gewebeschädigung ist als Dekubitus (Dekubitalgeschwür, Druckgeschwür) bekannt. Dekubitus tritt insbesondere bei pflegebedürftigen Menschen auf, die ans Bett gefesselt sind und bei denen bestimmte Körperpartien einer dauernden Druckbelastung ausgesetzt sind. Überschreitet der auf die Gefäße einwirkende Druck den Kapillardruck der Gefäße, so kommt es zu einer Unterversorgung der Zellen mit Sauerstoff und Nährstoffen und damit verbunden zu einer Schädigung des Gewebes. Während Druckgeschwüre bei gesunden Menschen in der Regel nicht auftreten, da diese sich regelmäßig umlagern und gefährdete Hautstellen entlasten, sind die entsprechenden Reflexe bei pflegebedürftigen Personen teilweise nur noch eingeschränkt vorhanden. Dekubitus kann insbesondere an solchen Hautstellen auftreten, an denen Knochen der Hautoberfläche besonders nahe kommen. Schließlich besteht auch die Gefahr, dass ein offenes Dekubitalgeschwür zum Eindringen von Keimen führt. Angesichts der Zahl der pflegebedürftigen Menschen und den erheblichen Konsequenzen beim Auftreten von Dekubitus besteht gerade für diese Indikation ein besonderer Bedarf nach Behandlungsmöglichkeiten.

Die Förderung der Wiederherstellung von verletztem Körpergewebe ist auch insofern vorteilhaft, als auf diese Weise der Ausbildung von Narben vorgebeugt werden kann. Es hat sich gezeigt, dass durch die erfindungsgemäße Zusammensetzung Wunden, insbesondere der Haut, besser verheilen und aus optischen Gründen unerwünschte Narben vermieden werden.

Die erfindungsgemäße Zusammensetzung ist insbesondere für die Verabreichung auf das verletzte Körpergewebe vorgesehen, d. h. die Anwendung erfolgt insbesondere lokal bzw. topisch. Entsprechend kann die Zusammensetzung als Salbe, Creme, Lotion, Milch, Flüssigkeit, Emulsion, Mikroemulsion, Spray, Suspension, Paste, Pulver oder als sonstiger Feststoff vorliegen.

Die Zusammensetzung kann übliche Hilfsstoffe enthalten, z. B. Trägermittel, Konservierungsmittel, Bakterizide, Lösungsvermittler, Vitamine, Stabilisatoren, Substanzen zum Verhindern des Schäumens, Verdickungsmittel, Farbstoffe, oberflächenaktive Substanzen, Emulgatoren, feuchthaltende Substanzen u.ä..

Salben, Pasten, Cremes und Gele können die üblichen Trägerstoffe enthalten, z. B. tierische und pflanzliche Fette, Wachse, Paraffine, Stärke, Traganth, Zellulosederivate, Polyethylenglykole, Silikone, Bentonite, Kieselsäure, Talkum und Zinkoxid oder Gemische dieser Stoffe.

Die Zusammensetzungen können weitere Wirkstoffe enthalten, es ist jedoch insbesondere auch möglich und für die Förderung der Wiederherstellung des Körpergewebes hinreichend, Zusammensetzungen zu verwenden, die lediglich Glucosylglycerol bzw. entsprechende Ester als Wirkstoff enthalten.

Möglich ist beispielsweise die Kombination von Glucosylglycerol bzw. entsprechenden Derivaten mit einem oder mehreren Wirkstoffen ausgewählt aus: Dexpanthenol oder Derivate, Arnica montana-Extrakt (Arnica), Capsaicin, Capsicum-Extrakt, Hypericum perforatum-Extrakt (Johanniskraut), Cardiospermum halicacabum (Ballonrebe, Herzsame), Hamamelis virginiana-Extrakt (Zaubernuss), Tocopherol, Allantoin, Bisabolol, Kakao-Extrakt, Silber, Nanosilber, Mikrosilber, amorphes Silber, Silber-Salze, Zink, Zinkoxid, Calendula officinalis-Extrakt (Ringelblume), Honig und Honigextrakte, Propolis, Melilotus officinalis-Extrakt, Beinwell-Extrakt (Symphytum), Echium vulgare-Extrakt, Cumin, Angelica sinensis-Extrakt, ferulic acid (Ferularäure), hyaluronic acid (Hyaluronsäure), Aloe Vera-Extrakt, Matricaria recutita (Kamille)-Extrakt, Allium cepa-Extrakt (Zwiebel), Achillea millefolium-Extrakt (Schafgarbe), Glycyrrhiza inflata-Extrakt (Süßholz), Licochalcon A, Silikon, Urea (Harnstoff), Echinacea purpurea (Sonnenhut)-Extrakt, Chichoriensäure.

Die Konzentration an Glucosylglycerol und/oder entsprechenden Estern kann insbesondere in einem Bereich zwischen 0,01 und 50 Gew.-%, insbesondere zwischen 0,5 und 20 Gew. % und besonders bevorzugt zwischen 1 und 10 Gew.-% liegen.

### Beispiel 3: Wundheilungsförderung durch Hydroxyectoin (nicht erfindungsgemäß) und Glucosylglycerol anhand von porcinen ex-vivo Hautstanzen

Es wurde ein porcines ex-vivo Wundheilungsmodell verwendet, wie es im Patent DE 103 17 400 B4 beschrieben wird.

**Proben:**

| | Konzentration (Gew.-%) | Osmolarität |
|---|---|---|
| Hydroxyectoin I | 3,84 | 302 |
| Hydroxyectoin hyperton | 6 | 454 |
| Glucosylglycerol I | 4,76 | 305 |
| Glucosylglycerol hyperton | 7,55 | 476 |
| PBS hyperton | | 463 |
| PBS isoton | | |

| | | |
|---|---|---|
| PBS: phosphatgepufferte Salzlösung | | |

Aus den Plicae von gewaschenen und sterilisierten Schweineohren wurden Stanzen mit einem Durchmesser von 6 mm entnommen. Aus der Mitte der Stanzen wurden in einem Bereich von 3 mm die Epidermis und die obere Dermis entfernt. Sofort nach Generierung der Modelle wurden in die Wunden 5 µl der Testsubstanzen gegeben, in die Kontrollmodelle wurden 5 µl PBS appliziert. Nach 48 h (t₄₈ₕ) wurden die Modelle schockgefroren und bei -80°C aufbewahrt.

Es wurden jeweils 14 Versuchsreihen durchgeführt, so dass für jede Probe 8 bis 11 auswertbare Modelle vorhanden waren.

Anfertigung von Schnitten aus den Modellen:
Die Modelle wurden komplett in Tissue Freezing Medium (Fa. Leica, Nussloch) eingebettet und mit dem Kryostaten 6 µm dünne Schnitte angefertigt. Dabei wurde darauf geachtet, dass man sich in der Mitte des jeweiligen Modells befand. Die Schnitte wurden auf SuperFrost Objektträger gegeben, luftgetrocknet, 10 min in -20°C kaltem Aceton fixiert und bei -80°C gelagert.

Sämtliche Modelle wurden mit Hämatoxylin/Eosin gefärbt (je 2 Schnitte). Der Ablauf der Färbung war wie folgt:

| | |
|---|---|
| 1) 6 min | Inkubation mit Hämatoxylin |
| 2) kurz | spülen in Leitungswasser |
| 3) kurz | spülen in HCl-Alkohol |
| 4) 15 min | spülen in Leitungswasser fließend |
| 5) kurz | spülen in Aqua dest |
| 6) 1 min | Inkubation in Eosin (0,2%) |
| 7) kurz | Spülen in Leitungswasser |
| 8) 20 sec | Inkubation in Aqua dest |
| 9) je 20 sec | Inkubation in aufsteigender Alkoholreihe (50%, 50%, 96%, 2 x 100%) |
| 10) 20 sec | Inkubation in Xylol |
| 11) anschließend: | Schnitte trocknen und mit Eukitt eindeckeln |

Die Färbungen wurden an einem Leica Lichtmikroskop DM LS und einer Olympus Camedia Digitalkamera ausgewertet.

Statistische Auswertungen (Wundheilungsscore):
0: kein Wundheilungsfortschritt
1: kleine Wundzunge
2: große Wundzunge
3: geschlossenes Sheet
4: mehrlagig geschlossenes Sheet

Es wurde, wenn beide Wundränder auswertbar waren, das Mittel zwischen linkem und rechtem Wundrand gebildet. Die statistischen Auswertungen wurden mit Hilfe des gepaarten Student-T-Tests durchgeführt.

### Auswertung:

1) Wundheilungsfortschritt absolut (alle auswertbaren aus 14 Modellen, d.h. ohne Modelle mit 2 Haarfollikeln in der Wunde oder großen Kratern), HE: Hydroxyectoin; GG: Glucosylglycerol; MW: Mittelwert; σ: Standardabweichung:

| | **MW** | **σ** | **SEM** |
|---|---|---|---|
| **HE I** | 1,92 | 0,96 | 0,30 |
| **HE hyperton** | 2,08 | 0,72 | 0,23 |
| **GG I** | 2,12 | 0,81 | 0,31 |
| **GG hyperton** | 1,66 | 0,93 | 0,30 |
| **PBS hyperton** | 1,60 | 0,84 | 0,28 |
| **PBS isoton** | 1,84 | 0,81 | 0,26 |

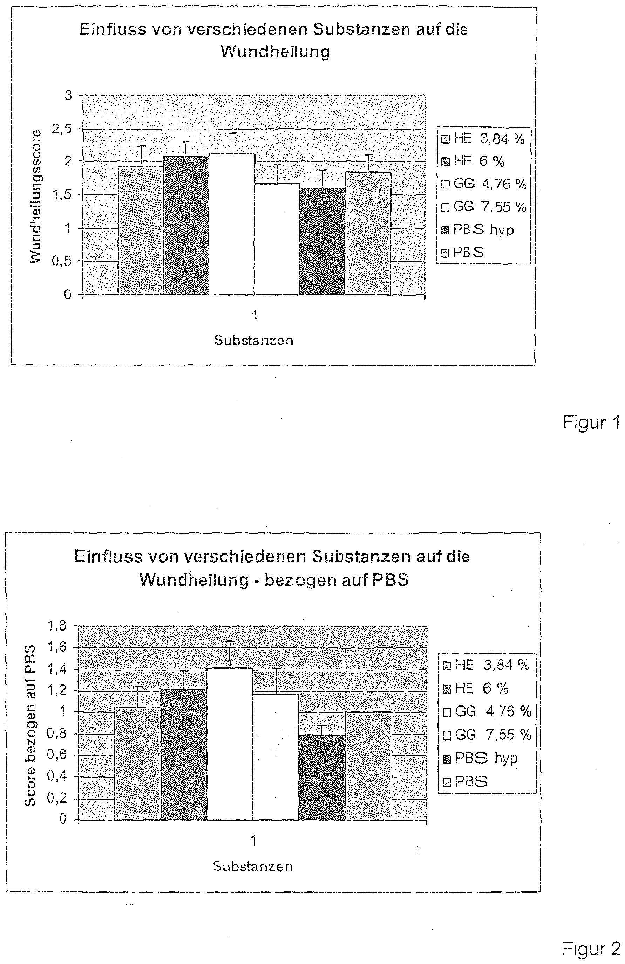

Figur 1 zeigt das Ergebnis in Form von Balkendiagrammen. Die Standardabweichungen werden als Striche über den Balken gezeigt (PBS = PBS isoton).
2) Wundheilungsfortschritt bezogen auf die PBS-Kontrolle; diese Auswertung erfolgt, um das Wundheilungspotential des einzelnen Schweins, das durch die mit PBS erzielten Werte ermittelt wird, zu berücksichtigen:

| | **MW** | **σ** | **SEM** |
|---|---|---|---|
| **HE I** | 1,05 | 0,62 | 0,20 |
| **HE hyperton** | 1,21 | 0,53 | 0,17 |
| **GG I** | 1,42 | 0,67 | 0,25 |
| **GG hyperton** | 1,17 | 0,78 | 0,25 |
| **PBS hyperton** | 0,78 | 0,30 | 0,10 |
| **PBS isoton** | 1,00 | 0 | 0 |

Figur 2 zeigt das Ergebnis in Form von Balkendiagrammen. Die Standardabweichungen werden als Striche über den Balken gezeigt (PBS = PBS isoton).

Sowohl Hydroxyectoin als auch Glucosylglycerol zeigen eine verbesserte Wundheilung.

## Patentansprüche

1. Zusammensetzung, enthaltend als Wirkstoff Glucosylglycerol und/oder Ester dieser Verbindung zur Verwendung in der Förderung der Wiederherstellung von verletztem Körpergewebe, wobei die Verletzung des Körpergewebes eine Wunde, das diabetische Fußsyndrom oder ein Ulcus ist, wobei es sich beim Ulcus um Ulcus cruris, Dekubitus, Malum perforans, Ulcus durum, Ulcus molle, Ulcus rodens oder Ulcus corneae handelt.

2. Zusammensetzung zur Verwendung nach Anspruch 1, **dadurch gekennzeichnet, dass** das Körpergewebe eine Haut oder Schleimhaut ist.

3. Zusammensetzung zur Verwendung nach Anspruch 1 oder 2, **dadurch gekennzeichnet, dass** die Verletzung des Körpergewebes eine thermische Wunde aufgrund von Verbrennungen, Verbrühungen oder Erfrierungen, eine Brandwunde, Verätzung oder eine auf ionisierende Strahlung zurückzuführende Wunde ist.

4. Zusammensetzung zur Verwendung nach Anspruch 1 oder 2, **dadurch gekennzeichnet, dass** die Verletzung des Körpergewebes eine Analfissur oder eine Hämorrhoidenverletzung ist.

5. Zusammensetzung zur Verwendung nach einem der Ansprüche 1 bis 4, **dadurch gekennzeichnet, dass** das Glucosylglycerol 2-O-α-Glucosylglycerol oder 2-O-β-Glucosylglycerol ist.

6. Zusammensetzung zur Verwendung nach Anspruch 5, **dadurch gekennzeichnet, dass** das Glucosylglycerol 2-O-α-D-Glucosylglycerol ist.

7. Zusammensetzung zur Verwendung nach einem der Ansprüche 1 bis 6, **dadurch gekennzeichnet, dass** die Zusammensetzung einen oder mehrere weitere Wirkstoffe enthält, ausgewählt aus: Dexpanthenol oder Derivate, Arnica montana-Extrakt (Arnica), Capsaicin, Capsicum-Extrakt, Hypericum perforatum-Extrakt (Johanniskraut), Cardiospermum halicacabum (Ballonrebe, Herzsame), Hamamelis virginiana-Extrakt (Zaubernuss), Tocopherol, Allantoin, Bisabolol, Kakao-Extrakt, Silber, Nanosilber, Mikrosilber, amorphes Silber, Silber-Salze, Zink, Zinkoxid, Calendula officinalis-Extrakt (Ringelblume), Honig und Honigextrakte, Propolis, Melilotus officinalis-Extrakt, Beinwell-Extrakt (Symphytum), Echium vulgare-Extrakt, Cumin, Angelica sinensis-Extrakt, ferulic acid (Ferularäure), hyaluronic acid (Hyaluronsäure), Aloe Vera-Extrakt, Matricaria recutita (Kamille)-Extrakt, Allium cepa-Extrakt (Zwiebel), Achillea millefolium-Extrakt (Schafgarbe), Glycyrrhiza inflata-Extrakt (Süßholz), Licochalcon A, Silikon, Urea (Harnstoff), Echinacea purpurea (Sonnenhut)-Extrakt, Chichoriensäure.

## Claims

1. Composition comprising as active ingredient glucosylglycerol and/or esters of this compound for use in promoting the regeneration of injured body tissue, wherein the injury to the body tissue is a wound, diabetic foot syndrome or an ulcer, wherein the ulcer is crural ulcer, decubitus, malum perforans, durum ulcer, molle ulcer, roden's ulcer or corneal ulcer.

2. Composition for use according to claim 1, **characterized in that** the body tissue is skin or mucous membrane.

3. Composition for use according to claim 1 or 2, **characterized in that** the injury of the body tissue is a thermal wound due to burns, scalds or frostbite, a burn, chemical burn or a wound due to ionizing radiation.

4. Composition for use according to claim 1 or 2, **characterized in that** the injury of the body tissue is an anal fissure or haemorrhoids injury.

5. Composition for use according to any of claims 1 to 4, **characterized in that** the glucosylglycerol is 2-O-α-glucosylglycerol or 2-O-β-glucosylglycerol.

6. Composition for use according to claim 5, **characterized in that** the glucosylglycerol is 2-O-α-D-glucosylglycerol.

7. Composition for use according to any of claims 1 to 6, **characterized in that** the composition comprises a substance or a plurality of further substances to be selected from: dexpanthenol or derivatives, arnica montana extract (arnica), capsaicin, capsicum extract, hypericum perforatum extract (St John's wort), cardiospermum halicacabum (balloon plant), hamamelis virginiana extract (witch hazel), tocopherol, allantoin, bisabolol, cocoa extract, silver, nanosilver, microsilver, amorphous silver, salts of silver, zinc, zinc oxide, calendula officinalis extract (marigold), honey and honey extracts, propolis, melilotus officinalis extract, comfrey extract (symphytum), echium vulgare extract, cumin, angelica sinensis extract, ferulic acid, hyaluronic acid, aloe vera extract, matricaria recutita (chamomile) extract, allium cepa (onion) bulb extract, achillea millefolium extract (yarrow), glycyrrhiza inflate extract (licorice), licochalcon A, silicone, urea, echinacea purpurea (purple coneflower) extract, chicoric acid.

## Revendications

1. Composition contenant en tant que principe actif du glucosylglycérol et/ou de l'ester dudit composé destinée à être utilisée pour promouvoir la reconstitution de tissus corporels blessés, dans laquelle la blessure des tissus corporels est une plaie, le syndrome du pied diabétique ou un ulcère, dans laquelle l'ulcère est un ulcère veineux, un décubitus, un mal perforant, une ulcération, un chancre mou, un carcinome basocellulaire ulcéré ou un ulcère cornéen.

2. Composition destinée à être utilisée selon la revendication 1, **caractérisée en ce que** les tissus corporels sont une peau ou une muqueuse.

3. Composition destinée à être utilisée selon la revendication 1 ou 2, **caractérisée en ce que** la blessure des tissus corporels est une plaie thermique liée à des brûlures ou des gelures, une brûlure, une brûlure chimique ou une plaie liée à un rayonnement ionisant.

4. Composition destinée à être utilisée selon la revendication 1 ou 2, **caractérisée en ce que** la blessure des tissus corporels est une fissure anale ou une blessure liée aux hémorroïdes.

5. Composition destinée à être utilisée selon l'une quelconque des revendications 1 à 4, **caractérisée en ce que** le glucosylglycérol est du 2-O-α-glucosylglycérol ou du 2-O-β-glucosylglycérol.

6. Composition destinée à être utilisée selon la revendication 5, **caractérisée en ce que** le glucosylglycérol est du 2-O-α-D-glucosylglycérol.

7. Composition destinée à être utilisée selon l'une quelconque des revendications 1 à 6, **caractérisée en ce que** la composition contient un ou plusieurs autres principes actifs, choisis parmi : dexpanthénol ou des dérivés, de l'extrait d'Arnica-montana (arnica), capsaïcine, extrait de capsicum, de l'extrait d'Hypericum perforatum (millepertuis), Cardiospermum halicacabum (plante ballon, pois de cœur), extrait d'Hamamelis virginiana (hamamélis), tocophérol, allantoïne, bisabolol, extrait de cacao, argent, nano-argent, microparticules d'argent, argent amorphe, sels d'argent, zinc, oxyde de zinc, extrait de Calendula officinalis (souci), miel et extraits de miel, propolis, extrait de Melilotus officinalis, extrait de consoude (Symphytum), extrait de Echium vulgare, cumin, extrait de Angelica sinensis, acide férulique, acide hyaluronique, extrait d'aloe vera, extrait de Matricaria recutita (camomille), extrait de Allium cepa (oignon), extrait d'Achillea millefolium (millefeuille), extrait de Glycyrrhiza inflata (réglisse), licochalcon A, silicone, urée, extrait d'Echinacea purpurea (échinacée pourpre), acide chichorique.
